# EUROPEAN PATENT SPECIFICATION

(11) **EP 1 414 368 B1**
(45) Date of publication and mention of the grant of the patent: **10.08.2016**
(21) Application number: 02736924.8
(22) Date of filing: 20.05.2002
(51) Int. Cl.: A61F 2/00, A01N 1/02, C12N 11/02, A61P 17/00, A61P 43/00

(54) **EB MATRIX PRODUCTION FROM FETAL TISSUES AND ITS USE FOR TISSUE REPAIR**
EB-MATRIX-PRODUKTION AUS FÖTALGEWEBE UND IHRE VERWENDUNG FÜR DIE GEWEBEREPARATUR
ELABORATION DE MATRICE DITE "EB" A PARTIR DE TISSU FOETAL, ET UTILISATION POUR LA REPARATION DES TISSUS

(30) Priority: 31.05.2001 US 871518
(43) Date of publication of application: 06.05.2004
(73) Proprietor: TEI Biosciences Inc., Boston, MA 02127 (US)
(72) Inventor: DAI, Jianwu, Boston, MA 02120 (US); BELL, Eugene, Boston, MA 02215 (US); RUSSAKOVSKY, Vladimir, Boston, MA 02132 (US)
(74) Representative: Lock, Graham James
(86) International application number: PCT/US2002/015620
(87) International publication number: WO 2002/096263

(56) References cited:
- EP-A- 0 742 018
- WO-A1-00/29037
- WO-A1-98/19718
- US-A- 4 801 299
- US-A- 5 624 463
- US-B1- 6 179 872
- SCOTCHFORD C A ET AL: "Osteoblast responses to collagen-PVA bioartificial polymers in vitro: the effects of cross-linking method and collagen content", BIOMATERIALS, ELSEVIER SCIENCE PUBLISHERS BV., BARKING, GB, vol. 19, no. 1-3, 1 January 1998 (1998-01-01), pages 1-11, XP027373132, ISSN: 0142-9612 [retrieved on 1998-01-01]

## Description

### BACKGROUND OF THE INVENTION

### FIELD OF THE INVENTION

This invention relates to the field of tissue engineering, and in particular to a method of processing animal tissue particularly, fetal or neo-natal tissue, to produce a biopolymer scaffold material named EB Matrix ("EBM"). EBM has broad applications for tissue repair and regeneration. It can serve as a remodelable scaffold for repair or replacement of human tissues and organs. It can be enriched with signaling molecules and cells before implantation, or with or without signaling molecules, it can attract host vessels and vascular cells as well as host parenchymal cells and immune systems cells to populate it after implantation. Also, it can serve as a delivery device for signaling factors, cells or drugs.

### DESCRIPTION OF THE RELATED ART

Rebuilding the human body is a significant industry. Human tissue banks and synthetic polymers do not meet the need for repair or replacement of body parts. High on the list of alternative sources of material used to meet this need are animal tissues prepared in new ways that reduce their immunogenicity and maximize their usefulness and efficacy.

In the field of tissue engineering, the following three components are used alone or in combination to repair or create new tissue and organ substitutes. 1) scaffolds made of naturally-occurring polymers (e.g. collagens), man-made polymers, (e.g. PTFE, Dacron, PET or polyethylene) or self-degrading, man-made polymers (e.g. PLA or PGA); 2) signaling molecules that give developmental instructions to cells; and 3) cells having specific or multiple tissue building potential, often referred to as "stem cells". Here we describe biopolymer matrices, produced by novel methods, from animal tissues including fetal and neo-natal tissues to be used as tissue engineering scaffolds.

Man-made implant materials such as synthetic polymers, plastics, and surface-coated metals may have different degrees of immunogenicity and suffer from significant limitations that prohibit their broad applications. A major limitation is that cells cannot remodel them after implantation. They are highly susceptible to microbial infection, and some undergo calcification. Synthetic vascular conduits have a high incidence of occlusion after peripheral vascular bypass procedures.

There is a long history of the use of biopolymer matrices made from processed human or animal tissues. Several methods of preserving collagen-based matrices from animal tissues have been developed (U.S. Patent No. 4,801,299, U.S. Patent No. 5,336,616, U.S. Patent No. 5,756,678, U.S. Patent No. 5,916,265 and U.S. Patent No. 5,997,895). All the methods include a chemical step that either kills or eliminates cells. Since tissues from post-natal animals or humans are the principal materials processed, a fixation step using glutaraldehyde or a similar agent may be used to mask antigenic determinants, eliminate the microbial burden and increase strength. However, aldehydic processing effectively destroys any biological activity, such as cell binding sites, associated with the original tissue and greatly reduces or eliminates the ability of cells to attach to it. It also eliminates binding sites for cell-synthesized products which attach to cells or to intermediates able to bind to cells and cell products that make up the extracellular matrix by cells.

Collagen-based devices that are animal-derived and fixed with glutaraldehyde or a similar agent can not be remodeled since they are highly resistant to metalloproteinase enzymes. Glutaraldehyde-treated devices are known to undergo gradual calcification. Heart valves made from fixed animal tissues can require replacement in 5-7 years or sooner due to calcification. The methods suggested in U.S. Patent No. 4,801,299 and U.S. Patent No. 5,916,265 include the use of glutaraldehyde or a similar agent for the fixation of tissue derived from a post-natal animal source. The resulting products can not be faithfully remodeled.

While detergents or sodium hydroxide may be used to process post-natal animal tissue (U.S. Patent No. 4,801,299, U.S. Patent No. 5,336,616, U.S. Patent No. 5,756,678, U.S. Patent No. 5,916,265, U.S. Patent No. 5,997,895), they have not been used to process fetal or neo-natal animal tissue. For example, U.S. Patent No. 5,997,895, filed on April 30, 1998, provides a certified collagen dural substitute derived from post-natal animal tissue that undergoes an alkaline/salt treatment involving sodium hydroxide and sodium sulfate (preferably in an aqueous solution of 5% sodium hydroxide and 20% sodium sulfate). A method for processing collagen containing materials which uses 1.0 N sodium hydroxide was disclosed in a journal article in 1989 by Diringer H. and Braig H.R. (Diringer H. and Braig H. R., 1989, Infectivity of unconventional viruses in dura mater. The Lancet, 439-440). This reference was cited in the FDA's Guide for 510(k) Review of Process Dura Mater (1990, 2).

The product of this invention, EBM, is unlike the other products cited above which in general are not bioremodelable. EBM is processed in a way that preserves its binding sites for cells and cell-secreted products that make up the extracellular matrix surrounding cells that occupy the scaffold. EBM is also distinguished by the fact that undesirable tissue components, such as DNA, are expressed mechanically from the tissue and that delipidyzing organic solvents are used to reduce the presence of cell and nuclear membranes. EBM does not calcify, making it safe for use in the human body for repair of soft tissues. In addition to its use for soft tissues, EBM can be used as a scaffold for bone repair if treated with an appropriate growth factor, if seeded with bone precursor cells or if occupied by bone forming cells when implanted.

EBM can be used as a tissue-building component with or without cells or signaling complexes for creating human body replacements. It can be used after the addition of signaling molecules, which will further promote tissue repair. It can also be implanted after stem or differentiated cells are seeded into or onto it.

### BRIEF SUMMARY OF THE INVENTION

By processing fetal or neo-natal animal tissueby the method embodying the invention, tissue strength is preserved without reducing its intrinsic biological properties or compromising the ability of cells that occupy the tissue to remodel it. In addition to chemical processing, a step of mechanically expressing undesirable tissue components from the tissue is a significant innovation. Additionally, the uniqueness of this invention is that it uses fetal or neo-natal tissue that, depending on age, is much less antigenic than adult tissue. The present invention overcomes the difficulties inherent in the approach to animal tissue use based on glutaraldehyde treatment.

### DETAILED DESCRIPTION OF THE INVENTION

For convenience, certain terms used in the specification, examples, and appended claims are collected here alphabetically.

The term "bioremodelable" or "bioremodelability" refers to a material that lends itself to the breakdown by cells that occupy it and use it as a template for creating a replacement made up mainly of newly synthesized components secreted by the cells.

The terms "delipidizing" or "delipidized" refers to the removal of lipids from the tissue.

The term "DHT" refers to a dehydrothermal process, wherein the tissue is crosslinked and dehydrated at a high temperature.

The term "drapability" includes the capacity of the material to mold to irregular, curved surfaces or surfaces of other geometries.

The terms "inactivating" or inactivated" refers to the reduction of the concentrations of infective agents (e.g. bacteria, molds, viruses and prions) by 4, 6 or 8 logs consistent with the requirements needed to insure against infectivity.

The phrase "mechanically expressing" refers to mechanically applying pressure to express undesirable components from the tissue. With the aid of appropriate solvents, unwanted components from the product that are potentially antigenic, such as DNA, RNA or other molecules released by reagents, such as NaOH, are removed.

The term "suturable" includes the ability to suture the material, wherein the material offers the required resistance to suture pull-out.

This invention involves preserving a naturally occurring, biopolymer-based matrix (EBM) from fetal or neo-natal animal tissue. It provides a method for producing and preserving a biopolymer scaffold material, comprising the steps of: a. treating an animal fetal tissue or neo-natal tissue harvested from a non-human animal source to inactivate infective agents of the tissue; b. mechanically applying pressure to the tissue to express undesirable components, including DNA, from the tissue; c. delipidizing the tissue; and d. washing the tissue for removal of chemical residues.

In a preferred embodiment, the fetal tissue is from a source between 10 weeks of age and newborn age.

In a further preferred embodiment, the method includes the step of extracting growth and differentiation factors from the tissue before treating the tissue to inactivate infective agents of the tissue, wherein the extracting step preferably comprises removing the growth and differentiation factors by buffer, enzyme or acid extraction.

The method according to the invention may include the step of drying the tissue and/or cross-linking the tissue.

In the preferred embodiment, porcine or bovine fetal and neo-natal tissues are used. Preferably, for example the fetal bovine tissue source is between 10 weeks of age and newborn age. As an example, fetal bovine skin is flash frozen and stored. Other source material includes blood vessels, other tubular structures, internal organs including the bladder, tendons, ligaments, cartilage, membranes such as the kidney capsule or diaphragm, or hard tissues such as cartilage or bone. After thawing, the tissue or organ is kept cold and chilled in an ice bath at a temperature between -4°C and 10°C. In a preferred embodiment, a salted ice bath is used to chill the tissue to a temperature below 0°C. The tissue adhering to the underside of the skin for example is mechanically removed.

Whereas, certain desirable naturally-occurring components of fetal and neo-natal tissues may be lost because of the harsh chemicals used for viral and prion inactivation and removal of unwanted structures and chemical components, some at high temperatures, desirable components such as growth and differentiation factors may be extracted from the skin or other tissue before the bleach and sodium hydroxide treatments for the purpose of viral and prion inactivation and for removal of unwanted structural and chemical components. In an alternative embodiment, growth and differentiation factors are extracted from the tissue by methods disclosed in U.S.

Application No. 60/251,125, filed on December 4, 2000 (e.g. buffer, enzyme or acid extraction). The extracted growth and differentiation factors, being in solution, are treated much more mildly with the agents used for viral and prion inactivation, such as with 1 N sodium hydroxide for 4 hrs. on a shaker on ice. After treatment, the extracted growth and differentiation factors are returned to the skin or other tissue being processed which readily absorbs them.

In the preferred embodiment, the skin undergoes microbial, fungal, viral and prion inactivation, beginning with a treatment with bleach. The bleach is at a concentration of between 0.05% and 5%, and the time of treatment can vary between 1 minute and 5 hours. This step can also be done after the sodium hydroxide step described below. All solutions are chilled with ice or salted ice to a temperature between -4°C and 10°C.

In the preferred embodiment, the tissue is washed extensively with water or physiological buffers (e.g. Tris-, HEPES, PBS buffer) to remove any residual bleach. The tissue is treated further with sodium hydroxide or potassium hydroxide at a concentration of between 0.1 N and 10 N for between 10 minutes and 2 hours. This treatment also inactivates infective agents of the tissue (e.g. bacteria, molds, viruses and prions). The container and all solutions are chilled with ice or salted ice to a temperature between -4°C and 10°C. The container is subsequently placed on a shaker.

In the preferred embodiment, unwanted components from the tissue (e.g. DNA, RNA or other molecules released by reagents such as NaOH) are mechanically expressed by means of repeated applications of pressure using a flat blade (like a putty knife) and/or roller(s). The steps of mechanically expressing material dissociated from the tissue chemically can be carried out by a machine as well, through an operation similar to that used manually.

Any organic solvent as well as mixtures of them suitable for dissolving lipids may be used for removal of lipid materials (e.g. chloroform, acetone, ether, alcohols and their mixtures). In the preferred embodiment, the tissue is delipidized in a chloroform and ethanol mixture (1:1 concentration ratio) for between 5 minutes and 5 hours followed by washes in 70% ethanol and water, or the foregoing solvents can be used *seriatim* with the ethanol being at a concentration of 70% for similar periods of time applied after the chloroform step.

In the preferred embodiment, the tissue is subjected to extensive washing with distilled water or buffers until the chemical residue is removed. The final product that is designated as EBM, is stored in distilled water or buffers, or dried.

In an alternative embodiment, EBM can be cross-linked with genipin or DHT. If DHT is used, the tissue undergoes dehydration at a high temperature.

In an alternative embodiment, EBM can be freeze-dried by rapidly freezing the tissue and then dehydrating it in a high vacuum. Freeze-drying increases the porosity and flexibility of the tissue.

This invention is further directed at an *in vitro* method for using a biopolymer scaffold material produced according to the method of the invention described above by applying the biopolymer scaffold material to lesion or to damaged tissue to promote tissue regeneration.

This invention is yet further directed at an *in vitro* method for using a biopolymer scaffold material, produced according to the method of the invention described above, as a cell delivery, signaling complex or drug delivery device by a. combining the biopolymer scaffold material with scaffolds made from naturally occurring, man-made or self-degrading polymers, or with signaling molecules or stem cells, or with drugs; wherein the signaling molecules comprise said growth and differentiation factors extracted from the tissue and treated with sodium hydroxide having a concentration consistent with the retention of biological activity; and b. applying the scaffold material and the scaffolds, signaling complexes, stem cells or drugs to lesion or to damaged tissue to promote tissue regeneration.

### Example 1

### Preparation of EBM

To prepare the preferred embodiment of EBM, the following protocol is followed. All of the following steps are performed in a laminar-flow hood using aseptic techniques and sterile solutions.

A piece of fetal bovine skin is cut out and a mark is made to distinguish the sides. The piece of skin is approximately 25x20 cm in size and is free of pigment or holes. The skin is dipped in a beaker containing 2.0 liters of Mili-Q™ water to rinse off excess blood. The skin is placed epidermal side down on a flat, plastic plate. The skin is flattened onto the plate.

Flesh is removed from the under side (dermal side) of the skin using dissection tools, if done by hand; serrated tipped forceps are used to lift the flesh, and curved scissors are used to remove approximately 5-10 mm wide continuous strips of flesh from one end of the skin to the other end. Defleshing, then removal of epidermis from the outer side of the skin can also be done by a defleshing machine. If carried out manually, the process should continue as follows.

The skin is placed in 20% bleach for 30±3 minutes on ice. A 1.0 liter square, wide-mouth bottle with a screw cap containing 500±50 mls of bleach provides approximately 1.0 ml of solvent per cm² of skin. The temperature of the bleach is 4±2 °C. The bottle is placed on a rocking platform. The skin is then washed in 2.0 liters of Mili-Q™ water for 20 minutes to dilute out the bleach.

The skin is placed on a flat, plastic plate epidermal side down. Any remaining subdermal flesh from the bottom side is removed with a flat blade like a putty knife by applying pressure to the blade as it is drawn over the surface of the tissue. This step mechanically expresses undesirable, chemically separated components from the tissue. The skin is held in place by a slip-resistant surface. The skin is turned epidermal side up and the epidermis is removed with a flat blade in strips of about 5.0 mm. A machine, through an operation similar to that used manually, can also perform the step of mechanically expression.

The skin is placed in a 5.2N solution of NaOH on a shaker on ice for 15±3 minutes. A 1.0 liter square, wide-mouth bottle with a screw cap containing 500±50 mls of NaOH provides approximately 1.0 ml of solvent per cm² of skin. The temperature of the NaOH is 4±2 °C.

The NaOH step is repeated twice with mechanical expression carried out between the steps and after the second step. The concentration of NaOH and the time of exposure to the NaOH can be increased or decreased depending upon the thickness of the skin. This is followed by three washes in 1.0 liter of Mili-Q™ water for up to 20 minutes each and additional steps of mechanical expression as needed.

As stated before, any organic solvent, as well as mixtures from them, can be used for removal of lipid material. In the preferred embodiment, a 1:1 chloroform ethanol solution treatment is followed by a 70% ethanol wash. This is followed by two washes in 1.0 liter of Mili-Q™ water for up to 10 minutes each with additional steps of mechanical expression as needed.

EBM is immersed in 1.0 liter of sterile PBS (phosphate buffered saline) for 20±4 hours on a rocking platform at a temperature of 4±2 °C which provides approximately 2.0 ml of PBS per cm² of skin.

EBM may be air-dried between two porous plates with or without the application of pressure for 24 hours. If increased porosity is desired, EBM can be freeze-dried.

### Example 2

### Use of EBM

EBM made from skin tissues can be used as a skin wound dressing or a skin replacement tissue. With or without the addition of signaling molecules and cells, EBM promotes wound healing. EBM is suitable for the treatment of chronic topical wounds such as burns, ulcers, and avulsion injuries. In grafts to host animals, such as rats, to replace full thickness skin wounds, acellular EBM is shown to remodel to replacement skin without scaring. However, secondary derivatives are absent. If seeded with dermal fibroblasts and keratinocytes, EMB can serve as a living skin replacement.

EBM can be used as a repair or replacement device throughout the human body. For example, EBM can be used as a urethral sling because of its high physical strength, resistance to stretch, suturability, cell-compatibility and bioremodelability.

EBM produced from fetal or neo-natal animal skins (e.g. porcine skin, bovine skin) can also be used for pericardial, periosteal, rotator cuff, or dura repair or replacement, or for hernia repair. It is drapable and has single suture-pullout strength of more than 20 Newtons.

EBM can be homogenized for use in an injectable form or as a foam in hemostats, dura replacement, and other similar areas of treatment. The same or similar processing described in the example above can be applied to non-skin tissues of the body to provide scaffolds of replacement parts with or without the addition of cells, signaling complexes or drugs, with the expectation that if acellular or cellular they will be vascularized and populated with host cells.

This disclosure is not limited to preserving EBM derived from those tissues or organs described herein. EBM may be derived from a wide variety of tissues and organs.

### EQUIVALENTS

Those skilled in the art will recognize, or be able to ascertain using no more than routine experimentation, numerous equivalents to the specific procedures described herein. Such equivalents are considered to be within the scope of the present invention and are covered by the following claims.

## Claims

1. A method for producing and preserving a biopolymer scaffold material, comprising the steps of:
a. treating an animal fetal tissue or neo-natal tissue that has been harvested from a non-human animal source to inactivate infective agents of the tissue;
b. mechanically applying pressure to the tissue to express undesirable components, including DNA, from the tissue;
c. delipidizing the tissue; and
d. washing the tissue for removal of chemical residues.

2. A method according to claim 1 wherein the fetal tissue is from a source between 10 weeks of age and newborn age.

3. A method according to claim 1 which includes the step of extracting growth and differentiation factors from the tissue before treating the tissue to inactivate infective agents of the tissue.

4. A method of claim 3, wherein the extracting comprises removing the growth and differentiation factors by buffer, enzyme or acid extraction.

5. A method according to any preceding claim which includes the step of drying the tissue.

6. A method according to any preceding claim which includes the step of cross-linking the tissue.

7. A method according to any preceding claim, wherein the step of treating the tissue to inactivate infective agents of the tissue comprises treating the tissue with sodium hydroxide or potassium hydroxide at a concentration between 0.1N and 10N.

8. A method according to claim 7, wherein the sodium hydroxide or potassium hydroxide treatment is applied for between 10 minutes and 2 hours.

9. A method according to claim 7 or claim 8, wherein the sodium hydroxide or potassium hydroxide treatment takes place at a temperature between -4°C and 10°C.

10. A method according to any preceding claim, wherein the step of mechanically applying pressure to the tissue includes repeated applications of pressure using a flat blade or roller(s).

11. A method according to any preceding claim wherein the step of treating the tissue to inactivate infective agents of the tissue comprises the steps of immersing the tissue in bleach for between 1 minute and 5 hours, wherein the bleach is at a concentration of between 0.05% and 5%, and chilling the tissue in an ice bath at a temperature between -4°C and 10°C, wherein salt is added to the ice bath to reach a temperature between 0°C and -4°C, and immersing the tissue in a solution of sodium hydroxide or potassium hydroxide for 10 minutes to 2 hours, wherein the solution is at a concentration of between 0.1N and 10N, and chilling the tissue in an ice bath at a temperature between -4°C and 10°C, wherein salt is added to the ice bath to reach a temperature between 0°C and -4°C.

12. A method according to any preceding claim wherein the step of delipidyzing comprises immersing the tissue in a chloroform and ethanol solution (1:1 concentration) for between 5 minutes and 5 hours, and washing the tissue in 70% ethanol and water.

13. A method according to claim 5, wherein the step of drying comprises freeze-drying or air-drying said tissue.

14. A method according to claim 6, wherein the step of cross-linking comprises cross-linking the tissue with genipin or DHT.

15. An in vitro method for using a biopolymer scaffold material produced in claim 1 by applying said biopolymer scaffold material to lesion or to damaged tissue to promote tissue regeneration.

16. An in vitro method for using a biopolymer scaffold material produced in claim 1 as a cell delivery, signaling complex or drug delivery device by
a. combining the biopolymer scaffold material with scaffolds made from naturally occurring, man-made or self-degrading polymers, or with signaling molecules or stem cells, or with drugs; wherein the signaling molecules comprise said growth and differentiation factors extracted from the tissue and treated with sodium hydroxide having a concentration consistent with the retention of biological activity; and
b. applying the scaffold material and the scaffolds, signaling complexes, stem cells or drugs to lesion or to damaged tissue to promote tissue regeneration.

## Patentansprüche

1. Verfahren zur Herstellung und Erhaltung eines Biopolymer-Gerüstmaterials, welches die Schritte umfasst:
a. Behandeln eines fötalen Tiergewebes oder neo-natalen Gewebes, welches aus einer nicht-menschlichen Quelle erhalten wurde, um infektiöse Mittel des Gewebes zu inaktivieren;
b. Ausüben mechanischen Drucks auf das Gewebe, um unerwünschte Komponenten, einschließlich DNA aus dem Gewebe herauszudrücken;
c. Entfernen von Lipiden aus dem Gewebe; und
d. Waschen des Gewebes zur Entfernung chemischer Reste.

2. Verfahren nach Anspruch 1, wobei das fötale Gewebe aus einer Quelle zwischen 10 Wochen und Neugeborenem ist.

3. Verfahren nach Anspruch 1, welches den Schritt umfasst, Extrahieren von Wachstums- und Differenzierungsfaktoren aus dem Gewebe vor Behandeln des Gewebes, um infektiöse Mittel des Gewebes zu inaktivieren.

4. Verfahren nach Anspruch 3, wobei das Extrahieren umfasst, Entfernen der Wachstums- und Differenzierungsfaktoren mittels Puffer-, Enzym- oder Säure-Extraktion.

5. Verfahren nach einem der vorstehenden Ansprüche, welches den Schritt umfasst, Trocknen des Gewebes.

6. Verfahren nach einem der vorstehenden Ansprüche, welches den Schritt umfasst, Vernetzen des Gewebes.

7. Verfahren nach einem der vorstehenden Ansprüche, wobei der Schritt des Behandelns des Gewebes zur Inaktivierung infektiöser Mittel des Gewebes umfasst, Behandeln des Gewebes mit Natriumhydroxid oder Kaliumhydroxid in einer Konzentration von zwischen 0,1 N und 10 N.

8. Verfahren nach Anspruch 7, wobei die Behandlung mit Natriumhydroxid oder Kaliumhydroxid für zwischen 10 Minuten und 2 Stunden durchgeführt wird.

9. Verfahren nach Anspruch 7 oder Anspruch 8, wobei die Behandlung mit Natriumhydroxid oder Kaliumhydroxid bei einer Temperatur zwischen - 4 °C und 10 °C erfolgt.

10. Verfahren nach einem der vorstehenden Ansprüche, wobei der Schritt der Ausübung mechanischen Drucks auf das Gewebe wiederholte Druckausübungen unter Verwendung eines flachen Blatts oder einer Rolle bzw. Rollen beinhaltet.

11. Verfahren nach einem der vorstehenden Ansprüche, wobei der Schritt des Behandelns des Gewebes zur Inaktivierung infektiöser Mittel des Gewebes die Schritte umfasst,
Eintauchen des Gewebes in Bleiche für zwischen 1 Minute und 5 Stunden, wobei die Bleiche in einer Konzentration von zwischen 0,05% und 5% vorliegt, und
Kühlen des Gewebes in einem Eisbad bei einer Temperatur zwischen -4°C und 10°C, wobei zu dem Eisbad Salz zugesetzt wird, um eine Temperatur von zwischen 0 °C und -4 °C zu erreichen, und
Eintauchen des Gewebes in eine Lösung von Natriumhydroxid oder Kaliumhydroxid für zwischen 10 Minuten und 2 Stunden, wobei die Lösung in einer Konzentration von zwischen 0,1 N und 10 N vorliegt, und
Kühlen des Gewebes in einem Eisbad bei einer Temperatur von zwischen - 4°C und 10°C, wobei zu dem Eisbad Salz zugesetzt wird, um eine Temperatur von zwischen 0 °C und -4 °C zu erreichen.

12. Verfahren nach einem der vorstehenden Ansprüche, wobei der Schritt der Entfernung von Lipiden umfasst, Eintauchen des Gewebes in eine Chloroform- und Ethanol-Lösung (1:1 Konzentration) für zwischen 5 Minuten und 5 Stunden, und Waschen des Gewebes in 70 % Ethanol und Wasser.

13. Verfahren nach Anspruch 5, wobei der Schritt des Trocknens umfasst, Gefriertrocknen oder Luft-Trocknen des Gewebes.

14. Verfahren nach Anspruch 6, wobei der Schritt der Vernetzung umfasst, Vernetzen des Gewebes mit Genipin oder DHT.

15. In vitro Verfahren zur Verwendung eines gemäß Anspruch 1 hergestellten Biopolymer-Gerüstmaterials durch Anwenden des Biopolymer-Gerüstmaterials auf eine Verletzung oder beschädigtes Gewebe zur Förderung der Geweberegenerierung.

16. In vitro Verfahren zur Verwendung eines gemäß Anspruch 1 hergestellten Biopolymer-Gerüstmaterials als Zell-Liefer-, Signalkomplex- oder Arzneimittel-Liefer-Einrichtung durch
a. Kombinieren des Biopolymer-Gerüstmaterials mit Gerüsten, die aus natürlich vorkommenden, vom Menschen hergestellten oder selbst-abbauenden Polymeren hergestellt wurden, oder mit Signalmolekülen oder Stammzellen, oder mit Arzneimitteln, wobei die Signalmoleküle die Wachstums- und Differenzierungsfaktoren enthalten, die aus dem Gewebe extrahiert wurden und mit Natriumhydroxid in einer mit der Erhaltung der biologischen Aktivität übereinstimmend Konzentration behandelt wurden; und
b. Anwenden des Gerüstmaterials und der Gerüste, Signalkomplexe, Stammzellen oder Arzneimittel bei Verletzungen oder beschädigtem Gewebe oder um die Geweberegenerierung zu fördern.

## Revendications

1. Procédé de production et de conservation d'un matériau de structure biopolymère, comprenant les étapes suivantes :
a. le traitement d'un tissu foetal ou tissu néo-natal animal provenant d'une source animale non humaine pour inactiver les agents infectieux du tissu ;
b. l'application d'une pression par voie mécanique au tissu pour extraire les composants indésirables, y compris l'ADN, du tissu ;
c. le dégraissage du tissu ; et
d. le lavage du tissu pour éliminer les résidus chimiques.

2. Procédé selon la revendication 1, dans lequel le tissu foetal provient d'une source dont l'âge est situé entre 10 semaines et l'âge d'un nouveau-né.

3. Procédé selon la revendication 1, qui comprend l'étape d'extraction des facteurs de croissance et de différenciation du tissu avant le traitement du tissu pour inactiver les agents infectieux du tissu.

4. Procédé selon la revendication 3, dans lequel l'extraction comprend l'élimination des facteurs de croissance et de différenciation par extraction avec un tampon, une enzyme ou un acide.

5. Procédé selon l'une quelconque des revendications précédentes, qui comprend l'étape de séchage du tissu.

6. Procédé selon l'une quelconque des revendications précédentes, qui comprend l'étape de réticulation du tissu.

7. Procédé selon l'une quelconque des revendications précédentes, dans lequel l'étape de traitement du tissu pour inactiver les agents infectieux du tissu comprend le traitement du tissu avec de l'hydroxyde de sodium ou de l'hydroxyde de potassium à une concentration située entre 0,1 N et 10 N.

8. Procédé selon la revendication 7, dans lequel le traitement avec l'hydroxyde de sodium ou l'hydroxyde de potassium est appliqué pendant 10 minutes à 2 heures.

9. Procédé selon la revendication 7 ou la revendication 8, dans lequel le traitement avec l'hydroxyde de sodium ou l'hydroxyde de potassium est réalisé à une température située entre -4 °C et 10 °C.

10. Procédé selon l'une quelconque des revendications précédentes, dans lequel l'étape d'application d'une pression par voie mécanique au tissu comprend des applications répétées de pression au moyen d'une lame plate ou de rouleau(x).

11. Procédé selon l'une quelconque des revendications précédentes, dans lequel l'étape de traitement du tissu pour inactiver les agents infectieux du tissu comprend les étapes d'immersion du tissu dans de l'eau de javel pendant 1 minute à 5 heures, dans lequel l'eau de Javel est à une concentration située entre 0,05 % et 5 %, et de refroidissement du tissu dans un bain de glace à une température située entre -4 °C et 10 °C, dans lequel du sel est ajouté au bain de glace pour atteindre une température située entre 0 °C et -4 °C, et d'immersion du tissu dans une solution d'hydroxyde de sodium ou d'hydroxyde de potassium pendant 10 minutes à 2 heures, dans lequel la solution est à une concentration située entre 0,1 N et 10 N, et de refroidissement du tissu dans un bain de glace à une température située entre -4 °C et 10 °C, dans lequel du sel est ajouté au bain de glace pour atteindre une température située entre 0 °C et -4 °C.

12. Procédé selon l'une quelconque des revendications précédentes, dans lequel l'étape de dégraissage comprend l'immersion du tissu dans une solution de chloroforme et d'éthanol (concentration 1/1) pendant 5 minutes à 5 heures, et le lavage du tissu dans de l'éthanol à 70 % et de l'eau.

13. Procédé selon la revendication 5, dans lequel l'étape de séchage comprend la lyophilisation ou le séchage à l'air dudit tissu.

14. Procédé selon la revendication 6, dans lequel l'étape de réticulation comprend la réticulation du tissu avec de la génipine ou du DHT.

15. Procédé *in* vitro d'utilisation d'un matériau de structure biopolymère produit selon la revendication 1, par l'application dudit matériau de structure biopolymère sur une lésion ou un tissu endommagé pour favoriser la régénération du tissu.

16. Procédé *in vitro* d'utilisation d'un matériau de structure biopolymère produit selon la revendication 1 comme dispositif de distribution de cellules, d'un complexe de signalisation ou de distribution de médicament par
a. la combinaison du matériau de structure biopolymère avec des supports préparés à partir de polymères d'origine naturelle, synthétiques ou autodégradables, ou avec des molécules de signalisation ou des cellules souches, ou avec des médicaments ; dans lequel les molécules de signalisation comprennent lesdits facteurs de croissance et de différenciation extraits du tissu et traités avec de l'hydroxyde de sodium ayant une concentration compatible avec la rétention de l'activité biologique ; et
b. l'application du matériau de structure et des supports, des complexes de signalisation, des cellules souches ou des médicaments sur une lésion ou un tissu endommagé pour favoriser la régénération du tissu.
